# EUROPÄISCHE PATENTSCHRIFT

(11) **EP 1 398 070 B1**
(45) Veröffentlichungstag und Bekanntmachung des Hinweises auf die Patenterteilung: **30.11.2005**
(21) Anmeldenummer: 03019117.5
(22) Anmeldetag: 23.08.2003
(51) Int. Cl.: B01D 61/36, B01D 53/22

(54) **Trennung von Stoffgemischen mittels Membranverfahren mit thermischer Konditionierung des Feedgemischs**
Separation of mixtures by a membrane process with thermal conditioning of the feed mixture
Séparation de mélanges par procédé membranaire avec conditionnement thermique du mélange à traiter

(30) Priorität: 03.09.2002 DE 10240604
(43) Veröffentlichungstag der Anmeldung: 17.03.2004
(73) Patentinhaber: BASF Aktiengesellschaft, 67056 Ludwigshafen (DE)
(72) Erfinder: Bitterlich, Stefan, Dr., 67246 Dirmstein (DE)

(56) Entgegenhaltungen:
- EP-A- 0 294 827
- US-A- 4 911 845
- US-A- 4 925 459
- PATENT ABSTRACTS OF JAPAN vol. 1995, no. 08, 29. September 1995 (1995-09-29) -& JP 07 124444 A (HITACHI ZOSEN CORP), 16. Mai 1995 (1995-05-16)
- PATENT ABSTRACTS OF JAPAN vol. 017, no. 247 (C-1059), 18. Mai 1993 (1993-05-18) -& JP 05 000226 A (UBE IND LTD), 8. Januar 1993 (1993-01-08)

## Beschreibung

Die vorliegende Erfindung betrifft ein verbessertes Verfahren zur Trennung von Stoffgemischen mittels einer oder mehrerer Membranen.

Membranverfahren zur Trennung gasförmig vorliegender Gemische, auch als "Gaspermeation" bezeichnet, sind dem Fachmann grundsätzlich bekannt. Beschreibungen finden sich u.a. in M. Mulder: "Basic Principles of Membrane Technology", 1991, Kluwer Academic Publishers und in R. Rautenbach "Membranverfahren, Grundlagen der Modul- und Anlagenauslegung", 1997, Springer Verlag.

Bei der Gaspermeation findet eine Trennung von zwei oder mehr Stoffen dadurch statt, dass die Durchlässigkeit der Membran für die Stoffe unterschiedlich groß ist. Häufig wird als Maß für die Durchlässigkeit die sog. Permeanz angegeben, die definiert ist als flächenspezifischer Fluss einer bestimmten Komponente, geteilt durch die transmembrane Differenz der Partialdrücke bzw. Fugazitäten dieser Komponente.

Bei den Membranen kann es sich um solche handeln, die nach dem sog. Lösungs-Diffusions-Mechanismus funktionieren, d.h. die Komponenten lösen sich in der Membran, z.B. einem Polymerfilm, diffundieren hindurch und desorbieren auf der anderen Seite wieder. Es kann sich aber auch um mikroporöse Membranen handeln, bei denen an Stelle der Lösung eine Adsorption auf der Innenseite der Poren, gefolgt von einer Oberflächendiffusion in den Poren stattfindet, oder um eine poröse Membran, die mittels Knudsen-Diffusion trennt.

Damit der beschriebene Diffusionsvorgang zu einem gerichteten Stofftransport über die Membran und damit zu einer Stofftrennung führt, muss für die zu trennenden Stoffe eine Differenz der transmembranen Partialdrücke vorliegen. Meistens wird diese erzeugt, indem eine ausreichend große transmembrane Differenz der Gesamtdrücke eingestellt wird. Dies kann z.B. bewerkstelligt werden, indem auf der Seite, auf der das zu trennende Gemisch aufgegeben wird, im folgenden als Feedseite bezeichnet, ein über dem Atmosphärendruck liegender Druck eingestellt wird und/oder auf der Seite, auf der das durch die Membran permeierte Gemisch abgezogen wird, im folgenden als Permeatseite bezeichnet, ein Vakuum erzeugt wird. Für viele Prozesse ist es vorteilhaft, die nötige Druckdifferenz durch einen geeigneten Überdruck auf der Feedseite herzustellen, u.a. weil so gegenüber einer Erzeugung der Partialdruckdifferenz allein durch Vakuum auf der Permeatseite höhere transmembrane Partialdruckdifferenzen möglich werden, was zu höheren Flüssen und somit einem niedrigeren Bedarf an Membranfläche für eine gegebene Trennaufgabe führt.

Es ist Stand der Technik, den besagten Überdruck durch eine mechanische Kompression des gasförmigen Feedgemischs, z.B. mittels Kolben-, Schrauben- oder Turbokompressoren zu erzeugen. Allerdings gestaltet sich die notwendige Druckerhöhung verfahrenstechnisch und wirtschaftlich aufwendig. Insbesondere die Kompression des Feedstromes in der Gasphase gestaltet sich wegen der Kompressibilität von Gasen deutlich aufwendiger als es im Vergleich dazu bei einer flüssigen Phase der Fall wäre. Weiterhin wird das Verfahren durch den ausschließlichen Einsatz der o.g. Apparate (Kolbenkompressoren etc.) zur Druckerhöhung zusätzlich verfahrenstechnisch und wirtschaftlich aufwendig.

In der US-A 4 925 459 wird ein einstufiges Membranverfahren zur Gaspermeation offenbart, wobei ein zunächst flüssiger Feedstrom mittels einer Pumpe und einem Verdampfer im Druck erhöht wird.

Gemäß der US-A 4 911 845 wird ein flüssiges Feedgemisch verdampft und der Dampf verdichtet zu einer Gasseparationsmembranstufe geleitet. Permeat und Retentat werden anschließend unter Nutzung der Abwärme zur Verdampfung des Feedgemisches kondensiert.

Es stellte sich somit die Aufgabe, ein Verfahren zu finden, welches die genannten Nachteile vermeidet und das eine verfahrenstechnisch einfache und wirtschaftlichere Methode zur Stofftrennung ermöglicht.

Demgemäß wurde ein Verfahren zur Trennung von Stoffgemischen mittels Gaspermeation unter Verwendung mehrerer Membranstufen gefunden, bei welchem man den Feedstrom vor Einleitung in die erste Membranstufe
a. in flüssiger Phase in seinem Druck erhöht und anschließend
b. den Feedstrom teilweise oder vollständig durch Wärmezufuhr verdampft,
   in der ersten Membranstufe den Feedstrom auftrennt, den so gewonnen Permeatstrom kondensiert und diesen neuen Feedstrom vor Einleitung in die zweite Membranstufe
c. in flüssiger Phase in seinem Druck erhöht und anschließend
d. Feedstrom teilweise oder vollständig durch Wärmezufuhr verdampft.

Gegenstand der vorliegenden Erfindung ist es, die unter erhöhtem Druck stehende gasförmige Feedmischung zu erzeugen durch eine Kombination aus Druckerhöhung in der zunächst flüssig vorliegenden Feedmischung mittels einer geeigneten, dem Fachmann bekannten Pumpe und nachfolgend Zufuhr von Wärme unter vollständiger oder teilweiser Verdampfung des zu trennenden Feedgemischs, wobei der Druck nach der Pumpe gleich dem an der Membranstufe einzustellenden Feeddruck plus dem sich zwischen der Pumpe und der Membranstufe ergebenden Druckverlust ist. Die Feedmischung wird gasförmig mit der Membranstufe in Kontakt gebracht.

Ein wesentlicher Vorteil dieses Verfahrens ist darin begründet, dass die für die Druckerhöhung eines gegebenen Stoffgemischs von einem gegebenen Druck p₁ auf einen Druck p₂ benötigte mechanische oder elektrische Energie deutlich niedriger ist, wenn das Stoffgemisch hierbei in flüssiger Phase vorliegt, wodurch die Wirtschaftlichkeit weiter gesteigert wird.

Die für die Erzeugung des Feeddruckes nötige Energie wird erfindungsgemäß zum größten Teil als thermische Energie eingebracht. Dies ist immer dann vorteilhaft, wenn thermische Energie deutlich kostengünstiger zur Verfügung steht als (für die Erzeugung von mechanischer Energie meistens notwendige) elektrische Energie. Bei vielen Prozessen, in denen eine Stofftrennung erforderlich ist, steht die hierfür benötigte thermische Energie praktisch kostenlos zur Verfügung, da sie z.B. als Abwärme aus chemischen Produktionsprozessen unmittelbar und effektiv genutzt werden kann.

Dabei beträgt der Anteil der thermisch eingebrachten Energie an der insgesamt zugeführten Energie zwischen etwa 90 %, und 99,99 %, bevorzugt zwischen 98 % und 99,9 %.

Die Druckerzeugung mittels Verdampfung ist besonders vorteilhaft anwendbar auf die Trennung von Gemischen, deren Komponenten kritische Temperaturen > 30°C, besser noch > 60°C aufweisen, weil dann das Vorliegen einer flüssigen Mischung der zu trennenden Stoffe, die ja Voraussetzung für den besagten Verdampfungsschritt ist, nicht das Vorhandensein einer kostspieligen Kälteanlage voraussetzt. Andererseits ist es vorteilhaft, wenn die beteiligten Stoffe Siedepunkte (bei Atmosphärendruck) unter 250°C, bevorzugt unter 200°C, aufweisen, weil ansonsten einerseits relativ viel thermische Energie für den Verdampfungsschritt benötigt wird und andererseits der Temperaturbereich, in dem die meisten Membranen funktionieren, verlassen wird.

Der auf die beschriebene Weise einzustellende Druck auf der Feedseite vor der Membran beträgt 1,5 bis 200, bevorzugt 3 bis 100, besonders bevorzugt 4 bis 60 bar abs. Der optimale Wert wird bestimmt durch die Zusammensetzung des zu trennenden Gemischs, die Trennaufgabe, die Eigenschaften der Membran und verfahrenstechnische und ökonomische Randbedingungen und kann vom Fachmann durch Routineversuche ermittelt werden.

Der Druck auf der Permeatseite liegt unter dem auf der Feedseite. Mit dieser Maßgabe ist er im Prinzip frei wählbar. Der optimale Wert wird auch hier bestimmt durch die Zusammensetzung des zu trennenden Gemischs, die Trennaufgabe, die Eigenschaften der Membran und verfahrenstechnische und ökonomische Randbedingungen. Das Permeat kann z.B. unter Atmosphärendruck oder unter Vakuum abströmen. Es kann aber auch vorteilhaft sein, den Permeatdruck auf einen über dem Atmosphärendruck liegenden Wert einzustellen, nämlich dann, wenn es verfahrenstechnische und/oder wirtschaftliche Vorteile bringt, das Permeat zu kondensieren und ein über Atmosphärendruck liegender Permeatdruck die Kondensation mit einem kostengünstigen Kühlmedium (z.B. Flusswasser) erlaubt.

Die Temperatur, bei der das zu trennende Gemisch mit der Membran in Kontakt gebracht wird, liegt zwischen etwa -20 und 300°C, bevorzugt 30 bis 250°C, besonders bevorzugt 50 bis 200°C.

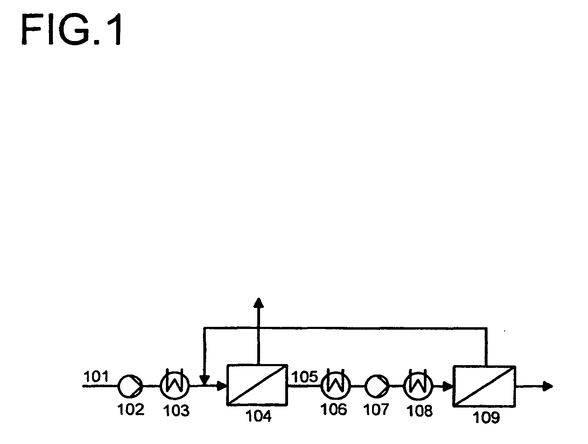

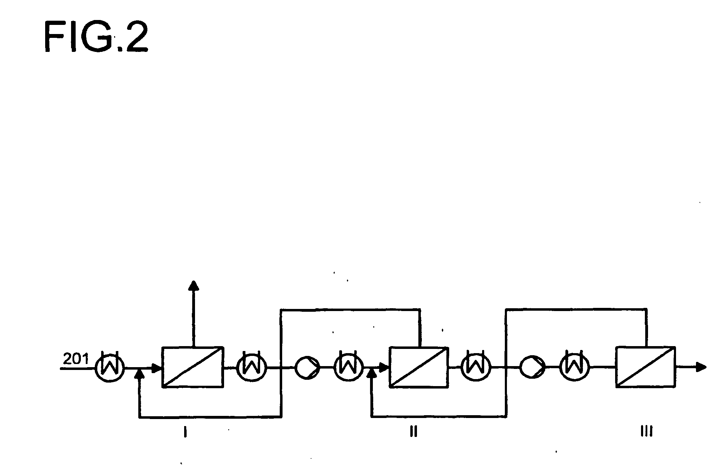

Die Vorteile des erfindungsgemäßen Verfahrens kommen besonders zum Tragen bei der Realisierung in an sich bekannten Verschaltungen mehrerer Membranstufen, bei denen aus einer Stufe das Permeat ganz oder teilweise als Feed in die jeweils folgende Stufe geführt wird und das Retentat ganz oder teilweise aus dieser Stufe dem Feed in die erstgenannte Stufe zugemischt wird (siehe z.B. Sep.Sci.Technol. 31 (1996), 729 ff), weil hier an einer oder mehreren Stellen die mechanische Kompression des gasförmigen Feedstromes vermieden wird. Exemplarisch sind 2 derartige Verschaltungen mehrerer Membranstufen in den Figuren 1 und 2 schematisch dargestellt, wobei hierbei auch die erfindungsgemäße Konditionierung der jeweiligen Feedgemische enthalten ist. In Figur 1 wird über der Feedstrom über Leitung (101) durch die Pumpe (102) in flüssiger Phase auf den gewünschten Druck gebracht und anschlieanschlieβend wird in Wärmetauscher (103) durch Wärmezufuhr der Feedstrom verdampft . In der Membran (104) erfolgt die Auftrennung des Feedstromes und der Permeatstrom wird über Leitung (105), einem Zwischenkühler (106) zur Kondensation zugeführt. Anschließend gelangt dieser "neue" Feedstrom über die Pumpe (107) und den Verdampfer (108) in die nächste Membran (109), in der die Trennung fortgeführt wird. Das bei der Membran (109) anfallende Retentat wird über Leitung (110) zu Leitung (101) kurz vor Eintritt in die Membran (104) zurückgeführt.

In Figur 2 ist die Verschaltung dreier Membranen exemplarisch dargestellt. Da der grundsätzliche Aufbau dem von Figur 1 ähnelt, wird dieser hier nicht näher beschrieben. Mit den Ziffern I,I I und III sind die hier vorliegenden 3 Membranstufen gekennzeichnet, innerhalb derer definitionsgemäß keine wesentliche Druckerhöhung durchgeführt wird. Es ist lediglich eine gewisse Druckdifferenz von Nöten, um den Transport zu gewährleisten.

Bei erfindungsgemäßen Anordnungen kann, abweichend von den Figuren, das zu trennende Gemisch (in Figur 2 ist dies der Strom 201) auch vor eine andere als die erste Membranstufe geführt werden.

Gleiches gilt für eine Verschaltung von 4 oder mehr Membranstufen im o.g. Sinne.

Dabei bezeichnet der Begriff Membranstufe eine Membraneinheit, innerhalb derer keine wesentliche Druckerhöhung durchgeführt wird. Sie kann einen oder mehrere Membranapparate aufweisen, wobei im letzteren Fall die der Membranstufe zuzurechnenden Membranapparate parallel von der jeweiligen Feedmischung angeströmt werden oder die aus einem Membranapparat oder mehreren parallel geschalteten Membranapparaten austretende Retentatmischung als Feed in einen Membranapparat oder mehrere parallel geschaltete Membranapparate geführt wird, was im Prinzip innerhalb einer Stufe beliebig oft, in der Praxis aber, bedingt durch den dabei auftretenden Druckverlust, maximal ca. 10 mal wiederholt werden kann.

Die erfindungsgemäße thermische Druckerzeugung lässt sich mit einer Vielzahl der dem Fachmann bekannten Membranen und Modulformen kombinieren.

Die verwendeten Membranen können z.B. aus Polymeren bestehen, oder aus anorganischem Material, das eine Trennung durch Knudsen-Diffusion bewirkt oder Molekularsieb-Eigenschaften besitzt, wie z.B. mikroporöser Kohlenstoff (durch Pyrolyse von organischen Polymeren wie z.B. PP hergestellt) oder Zeolithe. Die Membranen können als integralasymmetrische oder als Komposit-Membranen ausgeführt sein, bei denen die eigentliche die molekulare Trennung bewirkende Trennschicht, die eine Dicke von 0,1 bis 100, bevorzugt 1 bis 20 µm aufweist, auf einem oder mehreren meso- und/oder makroporösen Träger(n) aufgebracht ist.

Die Membranen kommen in Form von Flach-, Kissen-, Kapillar-, Monokanalrohr- oder Mehrkanalrohrelementen zum Einsatz, die dem Fachmann an sich v.a. aus anderen Membrantrennverfahren wie der Ultrafiltration oder Umkehrosmose bekannt sind (siehe z.B. R. Rautenbach "Membranverfahren, Grundlagen der Modul- und Anlagenauslegung", 1997, Springer Verlag). Bei Membranelementen mit Rohrgeometrie kann sich die Trennschicht vorzugsweise auf der Innen- oder Außenseite des Rohr befinden.

Die Membranen sind im allgemeinen umgeben von einem oder mehreren Gehäusen aus polymerem, metallischem oder keramischem Material, wobei die Verbindung zwischen Gehäuse und Membran durch ein abdichtendes Polymer (z.B. Elastomer) oder anorganisches Material gebildet wird.

Zur erfindungsgemäßen Einstellung des Feeddrucks kann der Fachmann auf an sich bekannte Pumpen, wie z.B. verschiedene Arten von Zentrifugalpumpen, aber auch durch Verdrängerpumpen wie Kolben-, Drehkolben-, Membran- oder Excenterschneckenpumpen zurückgreifen.

Der erfindungsgemäße Verdampfungsschritt kann erfolgen in beheizten Behältern mit Zuführung der Wärme durch die Wand oder eine im Behälter liegende Rohrschlange oder in einem oder mehreren ebenfalls dem Fachmann bekannten Wärmeübertragern, die z.B. Doppelrohr-, U-Rohr-, Rohrbündel- oder Plattengeometrie aufweisen können und im einfachen Durchgang oder als Natur- oder Zwangsumlaufverdampfer betrieben werden können. Es ist vorteilhaft, an geeigneter Stelle einen Abzug für schwersiedende Komponenten der Mischung vorzusehen, um deren Aufpegelung zu verhindern.

Das erfindungsgemäße Verfahren lässt sich besonders vorteilhaft anwenden auf Trennungen von Kohlenwasserstoffen mit 2 bis 10, bevorzugt 2 bis 6 Kohlenstoffatomen je Molekül, insbesondere auf die Trennungen von Mischungen von die überwiegend Komponenten mit gleicher Kohlenstoffzahl enthalten, z.B. die Trennung zwischen gesättigten und ungesättigten (siehe z.B. J. Membr.Sci. 184 (2001) 39 ff) oder die Trennung zwischen linearen und verzweigten Kohlenwasserstoffen (siehe z.B. Barri et al., EP 481659).

Das erfindungsgemäße Verfahren bietet eine verfahrenstechnisch einfache und wirtschaftliche Methode der Stofftrennung. Sie eignet sich besonders für Einsatzstoffe, deren Siedepunkt unter etwa 250°C liegt und ihre Vorteile kommen besonders bei der Verschaltung mehrerer Membranen zu Geltung.

## Patentansprüche

1. Verfahren zur Trennung von Stoffgemischen mittels Gaspermeation unter Verwendung mehrerer Membranstufen, **dadurch gekennzeichnet, dass** man den Feedstrom vor Einleitung in die erste Membranstufe
a. in flüssiger Phase in seinem Druck erhöht und anschließend
b. den Feedstrom teilweise oder vollständig durch Wärmezufuhr verdampft
in der ersten Membranstufe den Feedstrom auftrennt, den so gewonnen Permeatstrom kondensiert und diesen neuen Feedstrom vor Einleitung in die zweite Membranstufe
c. in flüssiger Phase in seinem Druck erhöht und anschließend
d. diesen Feedstrom teilweise oder vollständig durch Wärmezufuhr verdampft.

2. Verfahren nach Anspruch 1, **dadurch gekennzeichnet, dass** der Anteil der eingebrachten Energie, die durch Wärmezufuhr eingeleitet wird, zwischen 90 % und 99,9 % bezogen auf die insgesamt eingebrachte Energie beträgt.

3. Verfahren nach Ansprüchen 1 bis 2, **dadurch gekennzeichnet, dass** man auf der Feedseite der Membranstufen einen absoluten Druck von 1,5 bis 200 bar aufbaut.

4. Verfahren nach Ansprüchen 1 bis 3, **dadurch gekennzeichnet, dass** der Druck auf der Permeatseite der Membranstufen unterhalb dem Druck auf der Feedseite liegt.

5. Verfahren nach Ansprüchen 1 bis 4, **dadurch gekennzeichnet, dass** man das zu trennende Gemisch mit den Membranstufen bei einer Temperatur von -20°C bis 300°C in Kontakt bringt.

6. Verfahren nach Ansprüchen 1 bis 5, **dadurch gekennzeichnet, dass** man Gemische im Feedstrom einsetzt, deren Siedepunkte bei Atmosphärendruck unter 250°C liegen.

7. Verfahren nach Ansprüchen 1 bis 5, **dadurch gekennzeichnet, dass** man Gemische im Feedstrom zuführt, welche eine kritische Temperatur von mehr als 30°C aufweisen.

8. Verfahren nach Ansprüchen 1 bis 7, **dadurch gekennzeichnet, dass** man Gemische enthaltend Kohlenwasserstoffe mit 2 bis 10 Kohlenstoffatomen je Molekül auftrennt.

## Claims

1. A process for separating mixtures by means of gas permeation using two or more membrane stages, wherein the pressure of the feed stream before introduction into the first membrane stage
a. is increased in the liquid phase and then
b. some or all of the feed stream is vaporized by supplying of heat,
in the first membrane stage the feed stream is separated, the permeate stream thus obtained is condensed, and the pressure of this new feed stream, before introduction into the second membrane stage,
c. is increased in the liquid phase and then
d. some or all of this feed stream is vaporized by supplying of heat.

2. A process as claimed in claim 1, wherein that proportion of the energy provided which is introduced by heat supply is from 90 to 99.9%, based on the total energy provided.

3. A process as claimed in either of claims 1 and 2, wherein an absolute pressure from 1.5 to 200 bar is built up on the feed side of the membrane stage.

4. A process as claimed in any of claims 1 to 3, wherein the pressure on the permeate side is below the pressure on the feed side of the membrane stage.

5. A process as claimed in any of claims 1 to 4, wherein the mixture to be separated is brought into contact with the membrane stages at from -20°C to 300°C.

6. A process as claimed in any of claims 1 to 5, wherein mixtures whose boiling points are below 250°C at atmospheric pressure are used in the feed stream.

7. A process as claimed in any of claims 1 to 5, wherein mixtures which have a critical temperature of more than 30°C are fed in in the feed stream.

8. A process as claimed in any of claims 1 to 7, wherein mixtures containing hydrocarbons having 2 to 10 carbon atoms per molecule are separated.

## Revendications

1. Procédé de séparation de mélanges au moyen d'une perméation en phase gazeuse avec utilisation de plusieurs étapes à membrane, **caractérisé en ce que**, avant introduction du courant d'alimentation dans la première étape à membrane,
a. on augmente sa pression en phase liquide, et ensuite
b. on évapore partiellement ou totalement le courant d'alimentation par apport de chaleur,
on sépare le courant d'alimentation dans la première étape à membrane, on condense le courant de perméat ainsi obtenu et, avant introduction de ce nouveau courant d'alimentation dans la deuxième étape à membrane,
c. on augmente sa pression en phase liquide, et ensuite
d. on évapore partiellement ou totalement ce courant d'alimentation par apport de chaleur.

2. Procédé suivant la revendication 1, **caractérisé en ce que** la partie de l'énergie fournie, qui est introduite par apport de chaleur, représente entre 90 % et 99,9 % de l'énergie fournie au total.

3. Procédé suivant les revendications 1 et 2, **caractérisé en ce que**, du côté alimentation des étapes à membrane, on établit une pression absolue de 1,5 à 200 bars.

4. Procédé suivant les revendications 1 à 3, **caractérisé en ce que** la pression du côté perméat des étapes à membrane est inférieure à la pression du côté alimentation.

5. Procédé suivant les revendications 1 à 4, **caractérisé en ce qu'**on amène le mélange à séparer en contact avec les étapes à membrane à une température de -20°C à 300°C.

6. Procédé suivant les revendications 1 à 5, **caractérisé en ce qu'**on met en oeuvre dans un courant d'alimentation des mélanges dont les points d'ébullition sont inférieurs à 250°C à la pression atmosphérique.

7. Procédé suivant les revendications 1 à 5, **caractérisé en ce qu'**on amène dans le courant d'alimentation des mélanges qui présentent une température critique supérieure à 30°C.

8. Procédé suivant les revendications 1 à 7, **caractérisé en ce qu'**on sépare des mélanges contenant des hydrocarbures comportant de 2 à 10 atomes de carbone par molécule.
